# EUROPÄISCHE PATENTSCHRIFT

(11) **EP 0 420 815 B1**
(45) Veröffentlichungstag und Bekanntmachung des Hinweises auf die Patenterteilung: **19.01.1994**
(21) Anmeldenummer: 90810726.1
(22) Anmeldetag: 20.09.1990
(51) Int. Cl.: C07C 323/67, C07C 319/14

(54) **Verfahren zur Herstellung von 2-(2-Halogenethylthio)-phenylsulfonamiden**
Process for the preparation of 2-(2-haloethylthio)-phenylsulfonamides
Procédé pour la préparation de 2-(2-haloéthylthio)-phénylsulfonamides

(30) Priorität: 29.09.1989 CH 3554/89; 15.08.1990 CH 2654/90; 28.08.1990 CH 2786/90
(43) Veröffentlichungstag der Anmeldung: 03.04.1991
(73) Patentinhaber: CIBA-GEIGY AG, 4002 Basel (CH)
(72) Erfinder: Szczepanski, Henry, Dr., CH-4323 Wallbach (CH); Meyer, Willy, CH-4125 Riehen (CH); Weibel, Franz, Dr., CH-5264 Gipf-Oberfrick (CH)

(56) Entgegenhaltungen:
- EP-A- 0 044 808
- US-A- 4 141 916
- IL FARMACO, EDIZIONE SCIENTIFICA, Band 14, November 1959, Seiten 751-770, Pavia, IT; F. GALDI et al.: "Bis-amidi degli acidi fenil-ditio-benzen-2,2'-, 3,3'- e 4,4'- disolfonici"
- IL FARMACO, EDIZIONE SCIENTIFICA, Band 15, 1960, Seiten 835-841, Pavia, IT; F. GIALDI et al.: "Attivita' antifungina ed antibatterica di bis-amidi degli acidi fenilditiobezen-2,2'-3,3'-4,4'-disolfonici"

## Beschreibung

Die vorliegende Erfindung betrifft ein Verfahren zur Herstellung von 2-(2-Halogenethylthio)-phenylsulfonamiden der Formel I 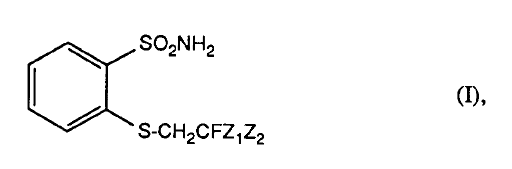
worin Z₁ und Z₂ unabhängig voneinander für Wasserstoff, Fluor oder Chlor stehen.

Die 2-(2-Halogenethylthio)-phenylsulfonamide der Formel I sind wertvolle Zwischenprodukte zur Herstellung von herbizid wirksamen N-Phenylsulfonyl-N'-pyrimidinyl- und -triazinylharnstoffen wie sie beispielweise in der europäischen Patentanmeldung Nr. 44808 offenbart sind.

Solche von den 2-(2-Halogenethylthio)-phenylsulfonamiden abgeleitete Phenylsulfonylharnstoffe zeichnen sich durch ein günstiges Abbauverhalten aus. Es besteht somit ein Bedarf nach einem vorteilhaften Herstellungsverfahren für das 2-(2-Halogenethylthio)-phenylsulfonamid der Formel I.

Es ist daher das Ziel der vorliegenden Erfindung, ein Verfahren bereitzustellen, welches die Herstellung von 2-(2-Halogenethylthio)-phenylsulfonamiden ausgehend von leicht zugänglichen Ausgangsstoffen auf einfache Weise und in guten Ausbeuten ermöglicht.

Es wurde nun gefunden, dass man die 2-(2-Halogenethylthio)-phenylsulfonamide der Formel I in vorteilhafter Weise herstellen kann, wenn man
a) ein 2-Halogenphenylsulfonamid der Formel II 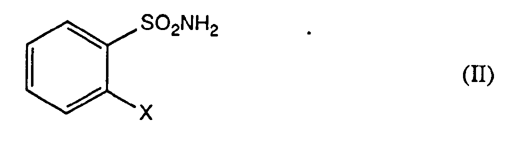 worin X für Fluor, Chlor oder Brom steht, in Gegenwart einer Base mit einem Mercaptan der Formel III

   R-SH (III)

   worin R für C₁-C₆-Alkyl oder durch Phenyl substituiertes C₁-C₆-Alkyl steht, zu einem 2-Sulfenylphenylsulfonamid der Formel IV 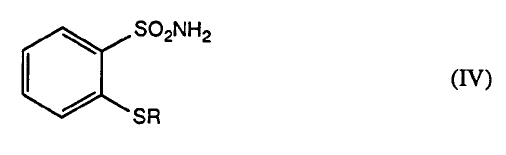 worin R die unter Formel III angegebene Bedeutung hat, überführt,
b) dieses zum 2-Sulfinyl-phenylsulfonamid der Formel V 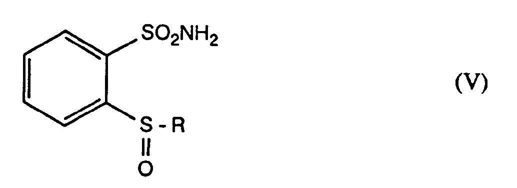 worin R die unter Formel III angegebene Bedeutung hat, oxidiert,
c) das erhaltene 2-Sulfinyl-phenylsulfonamid der Formel V in Gegenwart einer Säure zum Disulfid der Formel VI 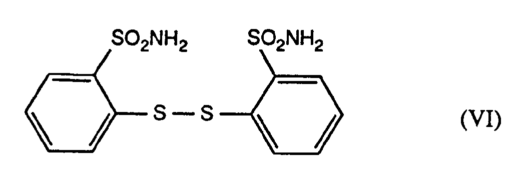 umsetzt,
d) das Disulfid der Formel VI zum 2-Mercapto-phenylsulfonamid der Formel VII 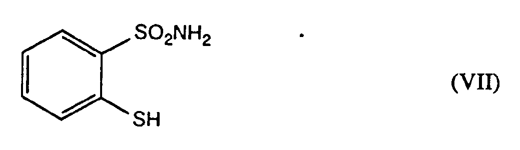 reduziert,
e) dieses anschliessend mit einem Trialkylamin der Formel X

   (R₁)₃N (X)

   worin R₁ für C₁-C₄-Alkyl steht, in das 2-Mercapto-phenylsulfonamid-Trialkylaminsalz der Formel VIII 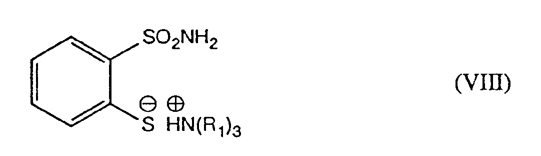 worin R₁ die unter Formel X genannte Bedeutung hat, überführt und
f) dieses anschliessend mit einem Halogen-fluorethan der Formel IX

   Y-CH₂CFZ₁Z₂ (IX)

   worin Y für Chlor oder Brom steht und Z1 und Z₂ unabhängig voneinander Wasserstoff, Fluor oder Chlor bedeuten, umsetzt.

Ausgangs- und Endprodukte des erfindungsgemässen Verfahrens sind bekannt.

Die Verbindungen der Formeln III, IV, V, VI, VII und IX sind bekannt und teilweise im Handel erhältlich.

Die 2-Mercapto-phenylsulfonamid-Trialkylaminsalze der Formel VIII sind neu und bilden ebenfalls einen Gegenstand der vorliegenden Erfindung.

Die im Substituenten R vorkommenden C₁-C₆-Alkylgruppen können geradkettig oder verzweigt sein und stehen beispielsweise für Methyl, Ethyl, n-Propyl, iso-Propyl, n-Butyl, sek.-Butyl, iso-Butyl, tert.-Butyl, n-Pentyl oder die isomeren Pentyl, n-Hexyl oder die isomeren Hexyl. Vorzugsweise besitzen die im Substituenten R vorkommenden Alkylgruppen 1 bis 3 Kohlenstoffatome. Sind die Alkylgruppen durch Phenyl substituiert, besitzen sie vorzugsweise eine Kettenlänge von 1 bis 3 Kohlenstoffatomen. Besonders bevorzugt steht der Substituent R für Benzyl.

Die in den Substituenten R₁ vorkommenden C₁-C₄-Alkylgruppen können geradkettig oder verzweigt sein und stehen für Methyl, Ethyl, n-Propyl, iso-Propyl, n-Butyl, sek.-Butyl oder tert.-Butyl. Besonders bevorzugt steht R₁ jeweils für Ethyl.

Der Verfahrensschritt a) des erfindungsgemässen Verfahrens wird mit Vorteil in einem inerten Lösungsmittel bei einer Temperatur zwischen +20°C und der Siedetemperatur des Lösungsmittels durchgeführt. Ueblicherweise liegen die Temperaturen zwischen +20 und +180°C, vorzugsweise zwischen +20 und +120°C. Ein besonders bevorzugter Temperaturbereich liegt zwischen +50 und +70°C.

Geeignete Lösungsmittel sind chlorierte Kohlenwasserstoffe, wie Dichlormethan, Trichlormethan, Trichlorethan oder Tetrachlorethan, Chlorbenzol oder Dichlorbenzol; aromatische Kohlenwasserstoffe wie Benzol, Toluol oder Xylol; Ether wie Diethylether, Diisopropylether, Tetrahydrofuran oder Dioxan; Nitrile wie Acetonitril oder Propionitril; Cyclohexan, Pyridin, N-Methylpyrrolidon oder N,N-Dimethylformamid wobei N,N-Dimethylformamid besonders bevorzugt ist.

Als Basen werden insbesondere Hydride, Hydroxide, Carbonate oder Alkoholate eines Alkali oder Erdalkalimetalls, ein Trialkylamin oder eine Pyridinbase verwendet. Besonders bevorzugte Basen sind Pyridin, Natriumhydroxid, Natriummethylat, Natriumethylat, Natriumcarbonat oder Kaliumcarbonat. Ganz besonders bevorzugt ist Kaliumcarbonat.

Der Verfahrensschritt a) kann in besonders vorteilhafter Weise durchgeführt werden, wenn man als 2-Halogenphenylsulfonamid der Formel II das 2-Fluor-phenylsulfonamid einsetzt.

Als Oxidationsmittel für den Verfahrensschritt b) sind insbesondere Permanganate, Perjodate, Persäuren oder Wasserstoffperoxid geeignet.

Bevorzugte Oxidationsmittel sind Peressigsäure, Perbenzoesäure, Perjodsäure, Kaliumpermanganat, Kaliumperiodat und Wasserstoffperoxid. Ein ganz besonders bevorzugtes Oxidationsmittel ist Wasserstoffperoxid. Die Oxidation des 2-Sulfenylphenylsulfonamids der Formel IV wird mit Vorteil bei Temperaturen von 0° bis +80°C durchgeführt, wobei ein Temperaturbereich von 0° bis +40°C bevorzugt wird.

In einer bevorzugten Variante des erfindungsgemässen Verfahrens wird die Oxidation des 2-Sulfenylphenylsulfonamids der Formel IV mit Wasserstoffperoxid in Gegenwart von Essigsäure bei einer Temperatur von +5 bis +15°C durchgeführt.

Der Verfahrensschritt c) wird vorzugsweise bei einer Temperatur von +20°C bis zur Siedetemperatur des Lösungsmittels durchgeführt. Als besonders gut geeignete Lösungsmittel haben sich Alkohole mit einer Kettenlänge von 1 bis 4 Kohlenstoffatomen erwiesen, wie zum Beispiel Methanol, Ethanol, Propanol, iso-Propanol, Butanol oder 2-Butanol. Ein bevorzugtes Lösungsmittel ist Methanol.

Säurekatalysierte Umlagerungen von Sulfoxiden sind in der Literatur unter dem Namen "Pummerer Umlagerungen" bekannt. Beispiele für derartige Reaktionen finden sich in Adv. Org. Chem. 6, 356 (1969). Im Gegensatz zu der aus diesem Artikel bekannten Lehre führt die säurekatalysierte Umlagerung des 2-Sulfinyl-phenylsulfonamids der Formel V nicht zum 2-Mercapto-phenylsulfonamid sondern überraschenderweise in guter Ausbeute und Reinheit zum 2,2'-Bis-aminosulfonyl-diphenyl-disulfid der Formel VI, welches aus seinem Reaktionsmedium auf einfache Weise isoliert werden kann und eine hohe Lagerstabilität aufweist.

Die Reaktion ist bezüglich der Natur der als Katalysatoren verwendeten Säuren unkritisch. Bevorzugte Säuren sind Mineralsäuren, insbesondere Salzsäure oder Schwefelsäure.

Die Verfahrensschritte b) und c) können auch unmittelbar nacheinander ohne Isolierung des Zwischenproduktes der Formel V in einem Reaktionsgefäss durchgeführt werden. Die für diese Verfahrensvariante geeigneten Lösungsmittel entsprechen den in Stufe c) genannten.

Die reduktive Spaltung des Disulfids der Formel VI zum 2-Mercapto-phenylsulfonamid der Formel VII (Verfahrensschritt d)) erfolgt im allgemeinen bei Temperaturen von +20°C bis +100°C.

Die Reduktion wird vorzugsweise mit Wasserstoff in Gegenwart von Edelmetallkatalysatoren, oder mit Zink, Eisen oder Zinn in Gegenwart von Salzsäure oder Essigsäure, oder mit Natrium-, Magnesium- oder Aluminiumamalgam durchgeführt. Bevorzugte Reduktionsmittel sind Wasserstoff in Gegenwart von Platin-, Palladium-, Rhodium- oder Nickelkatalysatoren sowie Zink, Eisen und Zinn in Gegenwart von Salzsäure oder Essigsäure. Ein ganz besonders bevorzugtes Reduktionsmittel ist Zink in Gegenwart von Salzsäure oder Essigsäure.

Durch die Ueberführung des 2-Mercaptophenylsulfonamids der Formel VII in das entsprechende Trialkylaminsalz der Formel VIII gemäss Verfahrensschritt e) wird sowohl die Reinheit als auch die Lagerfahigkeit dieses Zwischenprodukts erheblich verbessert. Ein für die Salzbildung gemäss Verfahrensschritt e) besonders gut geeignetes Trialkylarnin der Formel X ist das Triethylamin.

Die Reaktionstemperaturen bei der Umsetzung der 2-Mercapto-phenylsulfonamid-Trialkylaminsalze mit dem Halogen-fluorethan der Formel IX (Verfahrensschritt f)) liegen zwischen 0° und +80°C, bevorzugt zwischen 0° und +40°C. Die Reaktion verläuft besonders vorteilhaft, wenn in der Formel IX Y für Brom steht. Die für die Stufe f) geeigneten Lösungsmittel entsprechen den in Stufe a) genannten. Mit Vorteil werden diejenigen Verbindungen der Formel I hergestellt, in welcher Z₁ für Wasserstoff und Z₂ für Wasserstoff, Fluor oder Chlor steht. Bevorzugte Verbindungen der Formel IX sind 2-Brom-1-fluorethan, 2-Brom-1,1-difluorethan, 2-Brom-1-chlor-1-fluorethan und 1,2-Dichlor-1-fluorethan.

Das erfindungsgemässe Verfahren zeichnet sich durch zahlreiche vorteilhafte Eigenschaften aus. Die Reaktionsführung ist unkompliziert (Temperatur, Lösungsmittel, leichte Abtrennung der Zwischenprodukte, gute Entsorgung der Nebenprodukte und Rückstände etc.). Es kann mit oder ohne Isolierung des Zwischenprodukts der Formel V gearbeitet werden. Das Disulfid der Formel VI und die Trialkylaminsalze der Formel VIII sind besonders lagerstabil. Ausserdem weist das Verfahren in jeder einzelnen Reaktionsstufe eine hohe Ausbeute und hohe Produktqualität auf.

Das erfindungsgemässe Verfahren wird durch folgende Beispiele näher erläutert.

### Herstellungsbeispiele:

### Beispiel H1: Herstellung von 2-Benzylthio-phenylsulfonamid:

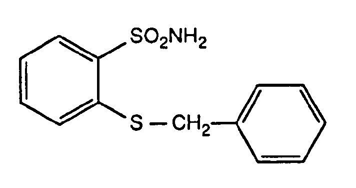

Zu einer Lösung von 124,2g Benzylmercaptan in 400 ml N,N-Dimethylformamid gibt man 175,2 g 2-Fluor-phenylsulfonamid und 160 g Kaliumcarbonat. Anschliessend wird die Reaktionsmischung für 3 Stunden auf +60°C erwärmt. Nach Abkühlen der Mischung auf +25°C wird filtriert und das Filtrat anschliessend eingedampft.

Der so erhaltene Rückstand wird mit 1500 ml Wasser versetzt, wobei das Produkt in Form farbloser Kristalle ausfällt. Nach Abtrennen der Lösung werden die erhaltenen Kristalle in Essigsäureethylester gelöst und anschliessend mit Magnesiumsulfat behandelt. Nach Abfiltrieren und Eindampfen des Filtrates erhält man 219 g (78,5% d. Th.) 2-Benzylthiophenylsulfonamid in Form farbloser Kristalle mit einem Schmelzpunkt von +104 bis +106°C.

### Beispiel H2: Herstellung von 2-Benzylsulfinyl-phenylsulfonamid:

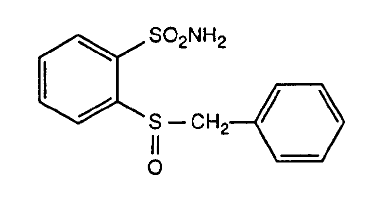

Zu einer Lösung von 219 g gemäss Beispiel H1 erhaltenen 2-Benzylthiophenylsulfonamids in 500 ml konzentrierter Essigsäure gibt man bei einer Temperatur von +10°C 102 ml 30%ige Wasserstoffperoxidlösung tropfenweise hinzu. Anschliessend wird die Reaktionsmischung bei einer Temperatur von +25°C 5 Stunden lang gerührt wobei das Produkt langsam auskristallisiert. Nach Abtrennen der Kristalle, Waschen mit Wasser und Trocknen erhält man 219 g (94,6 %) 2-Benzylsulfinyl-phenylsulfonamid in Form farbloser Kristalle mit einem Schmelzpunkt von +206 bis +209°C.

### Beispiel H3: Herstellung von 2,2'-Bis-aminosulfonyl-diphenyl-disulfid:

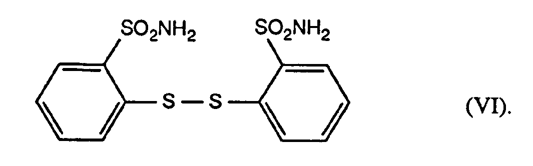

Zu einer Lösung aus 214 g eines gemäss Beispiel H2 hergestellten 2-Benzylsulfinyl-phenylsulfonamids in 500 ml Methanol gibt man 500 ml konzentrierte Salzsäure hinzu. Nach 7-stündigem Sieden der Reaktionsmischung und 2-tägigem Halten der Mischung bei Raumtemperatur wird das ausgefallene Disulfid abgetrennt und mit Wasser, Isopropanol und Diethylether gewaschen. Man erhält 133,2 g (97,7 % d. Th.) 2,2'-Bis-aminosulfonyldiphenyl-disulfid in Form gelblicher Kristalle mit einem Schmelzpunkt von +217°C (Zers.).

### Beispiel H4: Herstellung von 2,2'-Bis-aminosulfonyl-diphenyl-disulfid:

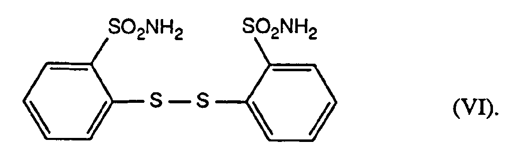

Zu einer Lösung von 10 g gemäss Beispiel H1 erhaltenen 2-Benzylthiophenylsulfonamids in 50 ml Ethanol gibt man tropfenweise 50 ml konzentrierte Salzsäure und 3,5 g 30%ige Wasserstoffperoxidlösung hinzu. Anschliessend wird die Reaktionsmischung für die Dauer von 1 Stunde auf Rückflusstemperatur erwärmt. Nach Abkühlen auf +25°C werden die entstandenen Kristalle abgetrennt und mit Isopropanol und Petrolether gewaschen. Man erhält 4 g 2,2'-Bisaminosulfonyl-diphenyldisulfid in Form gelber Kristalle mit einem Schmelpunkt von +211 bis +213°C.

### Beispiel H5: Herstellung von 2-Mercapto-phenylsulfonamid:

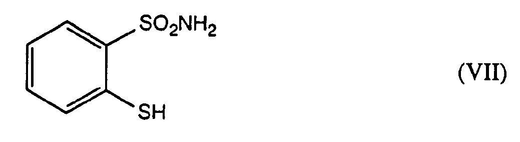

Eine Suspension aus 23,2 g eines gemäss Beispiel H3 und H4 erhaltenen 2,2'-Bis-aminosulfonyl-diphenyl-disulfids in 180 ml konzentrierter Essigsäure versetzt man mit 18 g Zinkpulver und erhitzt 30 Minuten auf Rückflusstemperatur. Anschliessend wird die Suspension auf +17°C gekühlt und filtriert. Nach Waschen des Filterrückstandes mit Essigsäureethylester wird das Filtrat eingedampft und der Rückstand anschliessend in 200 ml Essigsäureethylester gelöst. Nach zweimaligem Waschen mit 100 ml Wasser und Trocknen mit Magnesiumsulfat wird die Lösung eingedampft. Man erhält 22 g (94% d. Th.) ungereinigtes 2-Mercapto-phenylsulfonamid mit einem Schmelzpunkt von +113 bis +140°C.

### Beispiel H6: Herstellung von 2-Mercapto-phenylsulfonamid-Triethylaminsalz:

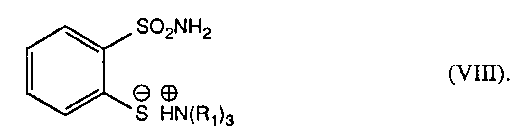

Zu einer Lösung von 7,6 g gemäss Beispiel H5 erhaltenen 2-Mercaptosulfonamids in 60 ml Tetrahydrofuran gibt man bei einer Temperatur von +25°C tropfenweise 5 g Triethylamin hinzu. Anschliessend werden die ausgefallenen, farblosen Kristalle abgetrennt und mit Diethylether gewaschen. Man erhält 9,2 g 2-Mercapto-phenylsulfonamid-Triethylaminsalz mit einem Schmelzpunkt von +164 bis +168°C.

### Beispiel H7: Herstellung von 2-(2-Fluorethylthio)-phenylsulfonamid:

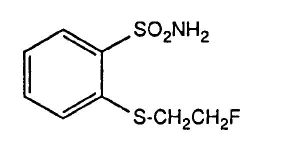

Eine Mischung aus 5,8 g das gemäss Beispiel H6 erhaltenen 2-Mercaptophenylsulfonamid-Triethylaminsalzes und 1,7 g 2-Chlor-1-fluorethan in 40 ml Methanol lässt man bei einer Temperatur von +50 bis +55°C 23 Stunden lang in einem Einschlussrohr rühren. Anschliessend wird die Reaktionsmischung eingedampft und der verbliebene Rückstand mit Wasser versetzt. Nach Extrahieren mit Essigsäureethylester wird die organische Phase gewaschen und getrocknet. Nach Eindampfen der Lösung erhält man 3,5 g 2-(2-Fluorethylthio)-phenylsulfonamid (74,5 % d. Th.) mit einem Schmelzpunkt von +83 bis +85°C.

### Beispiel H8: Herstellung von 2-(2-Fluorethylthio)-phenylsulfonamid:

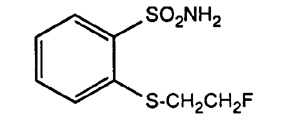

Eine Mischung aus 29 g des gemäss Beispiel H6 erhaltenen 2-Mercaptophenylsulfonamid-Triethylaminsalzes und 2 g 2-Brom-1-fluorethan in 130 ml Tetrahydrofuran lässt man bei einer Temperatur von +40°C bis +45°C 6 Stunden lang rühren. Anschliessend kühlt man die Reaktionsmischung auf 0°C ab und filtriert. Nach Eindampfen des Filtrates und Kristallisieren des erhaltenen Rückstandes aus Dichlormethan erhält man 21,2 g (90,2 % d. Th.) 2-(2-Fluorethylthio)-phenylsulfonamid mit einem Schmelzpunkt von +84 bis +85°C.

### Beispiel H9: Herstellung von 2-(2,2-Difluorethylthio)-phenyl-sulfonamid:

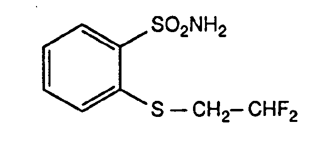

Eine Mischung aus 145,2 g des gemäss Beispiel H6 erhaltenen 2-Mercapto-phenylsulfonamid-Triethylaminsalzes und 79,7 g 2-Brom-1,1-difluorethan in 600 ml Methanol lässt man bei einer Temperatur von +55°C bis +60°C 16 Stunden lang rühren. Anschliessend wird die Reaktionsmischung eingedampft. Durch Verrühren des Rückstandes mit Eiswasser und Filtrieren der so erhaltenen Suspension erhält man 120 g (95% d. Th.) 2-(2,2-Difluorethylthio)-phenyl-sulfonamid mit einem Schmelzpunkt von +111°C bis +112°C.

### Beispiel H10: Herstellung von 2-(2-Chlor-2-fluorethylthio)-phenyl-sulfonamid:

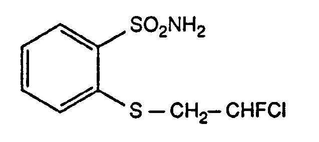

Eine Mischung aus 217,8 g des gemäss Beispiel H6 erhaltenen 2-Mercapto-phenylsulfonamid-Triethylaminsalzes und 101 g 1,2-Dichlor-1-fluorethan in 950 ml Methanol lässt man bei einer Temperatur von +65°C bis +70°C 24 Stunden und bei +95°C bis +100°C weitere 24 Stunden in einem Autoklaven rühren. Anschliessend wird die Reaktionsmischung eingedampft und der Rückstand mit Wasser verrührt. Durch Extrahieren mit Essigsäureethylester, Waschen mit Wasser, Trocknen über Natriumsulfat, Eindampfen und chromatographischer Reinigung des Rückstandes mit Methylenchlorid erhält man 99,5 g (49,2 % d. Th.) 2-(2-Chlor-2-fluorethylthio)-phenyl-sulfonamid mit einem Schmelzpunkt von +88°C bis +89°C.

## Patentansprüche

1. Verfahren zur Herstellung von 2-(2-Halogenethylthio)-phenylsulfonamid der Formel I 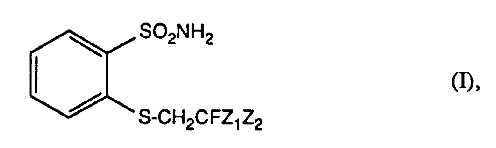 worin Z₁ und Z₂ unabhängig voneinander für Wasserstoff, Fluor oder Chlor stehen, dadurch gekennzeichnet, dass man
a) ein 2-Halogenphenylsulfonamid der Formel II 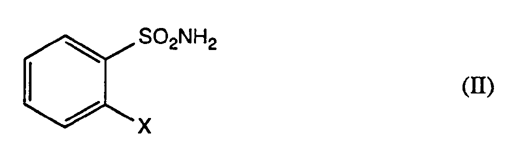 worin X für Fluor, Chlor oder Brom steht, in Gegenwart einer Base mit einem Mercaptan der Formel III
R - SH (III)
worin R für C₁-C₆-Alkyl oder durch Phenyl substituiertes C₁-C₆-Alkyl steht, zu einem 2-Sulfenylphenylsulfonamid der Formel IV 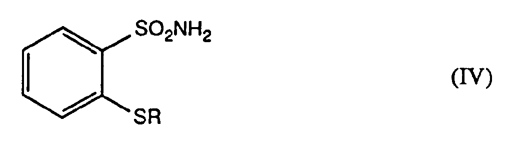 worin R die unter Formel III angegebene Bedeutung hat, überführt,
b) dieses zum 2-Sulfinyl-phenylsulfonamid der Formel V 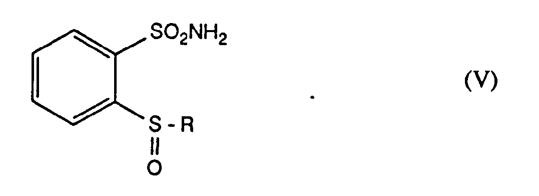 worin R die unter Formel III angegebene Bedeutung hat, oxidiert,
c) das erhaltene 2-Sulfinyl-phenylsulfonamid der Formel V in Gegenwart einer Säure zum Disulfid der Formel VI 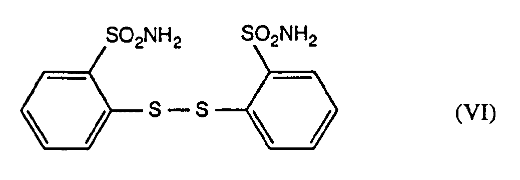 umsetzt,
d) das Disulfid der Formel VI zum 2-Mercapto-phenylsulfonamid der Formel VII 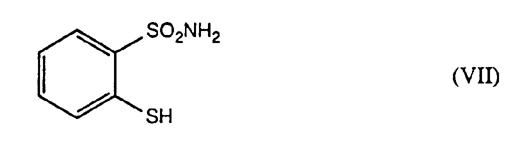 reduziert,
e) dieses anschliessend mit einem Trialkylamin der Formel X
(R₁)₃N (X)
worin R₁ für C₁-C₄-Alkyl steht, in das 2-Mercapto-phenylsulfonamid-Trialkylaminsalz der Formel VIII 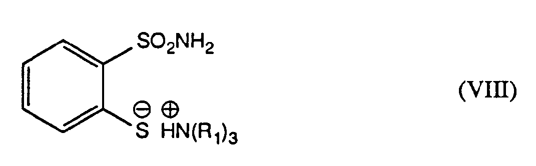 worin R₁ die unter Formel X genannte Bedeutung hat, überführt und
f) dieses anschliessend mit einem Halogen-fluorethan der Formel IX
Y-CH₂CFZ₁Z₂ (IX)
worin Y für Chlor oder Brom steht und Z₁ und Z₂ unabhängig voneinander Wasserstoff, Fluor oder Chlor bedeuten, umsetzt.

2. Verfahren nach Anspruch 1, dadurch gekennzeichnet, dass man als Trialkylamin der Formel X Triethylamin verwendet.

3. Verfahren nach Anspruch 1, dadurch gekennzeichnet, dass X für Fluor steht.

4. Verfahren nach Anspruch 1, dadurch gekennzeichnet, dass R für C₁-C₃-Alkyl oder durch Phenyl substituiertes C₁-C₃-Alkyl steht.

5. Verfahren nach Anspruch 3, dadurch gekennzeichnet, dass R durch Phenyl substituiertes C₁-C₃-Alkyl bedeutet.

6. Verfahren nach Anspruch 4, dadurch gekennzeichnet, dass R für Benzyl steht.

7. Verfahren nach Anspruch 1, dadurch gekennzeichnet, dass man bei der Umsetzung der Verbindung der Formel II mit der Verbindung der Formel III als Base ein Hydrid, Hydroxid, Carbonat oder Alkoholat eines Alkali- oder Erdalkalimetalls, ein Trialkylamin oder eine Pyridinbase verwendet.

8. Verfahren nach Anspruch 7, dadurch gekennzeichnet, dass man als Base Pyridin, Natriumhydroxid, Natriummethylat, Natriumethylat, Natriumcarbonat oder Kaliumcarbonat verwendet.

9. Verfahren nach Anspruch 7, dadurch gekennzeichnet, dass man als Base Kaliumcarbonat verwendet.

10. Verfahren nach Anspruch 1, dadurch gekennzeichnet, dass man für die Umsetzung der Verbindung der Formel IV in die Verbindung der Formel V als Oxidationsmittel Wasserstoffperoxid, Peressigsäure, Perbenzoesäure, Perjodsäure, Kaliumpermanganat oder Kaliumperjodat verwendet.

11. Verfahren nach Anspruch 10, dadurch gekennzeichnet, dass man als Oxidationsmittel Wasserstoffperoxid verwendet.

12. Verfahren nach Anspruch 1, dadurch gekennzeichnet, dass man zur Ueberführung des Disulfids der Formel VI in das 2-Mercapto-phenylsulfonamid der Formel VII als Reduktionsmittel Wasserstoff in Gegenwart von Platin-, Palladium-, Rhodium- oder Nickelkatalysatoren, oder Zink, Eisen oder Zinn in Gegenwart von Salzsäure oder Essigsäure verwendet.

13. Verfahren nach Anspruch 12, dadurch gekennzeichnet, dass man als Reduktionsmittel Zink in Gegenwart von Salzsäure oder Essigsäure verwendet.

14. Verfahren nach Anspruch 1, dadurch gekennzeichnet, dass man das 2-Sulfenylsulfonamid der Formel IV ohne Isolierung des Zwischenproduktes der Formel V direkt in das Disulfid der Formel VI überführt.

15. Verfahren nach Anspruch 1, dadurch gekennzeichnet, dass Y für Brom steht.

16. Verfahren nach Anspruch 1, dadurch gekennzeichnet, dass man die Umsetzung des 2-Halogenphenylsulfonamids der Formel II mit dem Mercaptan der Formel III bei einer Temperatur von +20 bis +120°C durchführt.

17. Verfahren nach Anspruch 1, dadurch gekennzeichnet, dass man die Oxidation des 2-Sulfenylphenylsulfonamids der Formel IV bei einer Temperatur von 0° bis 40°C durchführt.

18. Verfahren nach Anspruch 1, dadurch gekennzeichnet, dass man die Umsetzung des 2-Mercaptophenylsulfonamid-trialkylaminsalzes der Formel VIII mit dem 2-Halogenfluorethan der Formel IX bei einer Temperatur von 0° bis 80°C durchführt.

19. Verfahren nach Anspruch 1, dadurch gekennzeichnet, dass Z₁für Wasserstoff und Z₂ für Wasserstoff, Fluor oder Chlor steht.

20. Verfahren nach Anspruch 1, dadurch gekennzeichnet, dass man a) ein 2-Fluorphenylsulfonamid der Formel II in Gegenwart einer Base bei einer Temperatur von +50 bis +70°C mit einem Benzylmercaptan der Formel III zum 2-Benzylthio-phenylsulfonamid der Formel IV überführt,
b) dieses bei einer Temperatur von +5 bis +15°C mit Wasserstoffperoxid zum 2-Benzylsulfinyl-phenylsulfonamid der Formel V oxidiert,
c) dieses in Gegenwart von Salzsäure oder Schwefelsäure zum Disulfid der Formel VI umsetzt,
d) das Disulfid der Formel VI mit Zink in Gegenwart von Essigsäure zum 2-Mercapto-phenylsulfonamid der Formel VII reduziert,
e) dieses mit Triethylamin zum 2-Mercapto-phenylsulfonamid-Triethylaminsalz der Formel VIII überführt und
f) dieses anschliessend mit 2-Brom-1-fluorethan, 2-Brom-1,1-difluorethan, 2-Brom-1-chlor-1-fluorethan oder 1,2-Dichlor-1-fluorethan umsetzt.

21. Verfahren nach Anspruch 20, dadurch gekennzeichnet, dass man als Base Pyridin, Natriumhydroxid, Natriummethylat, Natriumethylat, Natriumcarbonat oder Kaliumcarbonat verwendet.

22. Verfahren nach Anspruch 21, dadurch gekennzeichnet, dass man als Base Kaliumcarbonat verwendet.

23. Verfahren nach Anspruch 21, dadurch gekennzeichnet, dass man das 2-Benzylthio-phenylsulfonamid der Formel IV ohne Isolierung des Zwischenprodukts der Formel V in Gegenwart von Salzsäure und Wasserstoffperoxid in das Disulfid der Formel VI überführt.

24. 2-Mercapto-phenylsulfonamid-Trialkylaminsalze der Formel VIII

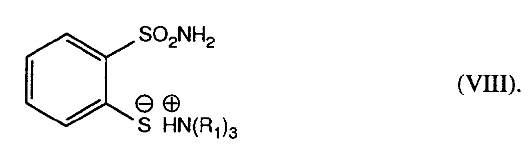

25. Verbindung der Formel VIII nach Anspruch 24, dadurch gekennzeichnet, dass R₁ jeweils für Ethyl steht.

## Claims

1. A process for the preparation of a 2-(2-halogenoethylthio)-phenylsulfonamide of the formula I 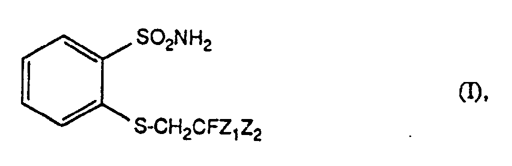 in which Z₁ and Z₂ independently of one another are hydrogen, fluorine or chlorine, wherein
a) a 2-halogenophenylsulfonamide of the formula II 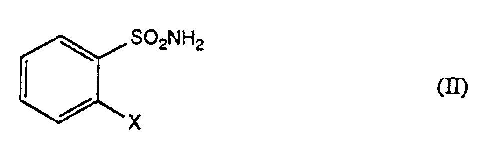 in which X is fluorine, chlorine or bromine, is converted, in the presence of a base, together with a mercaptan of the formula III
R-SH (III)
in which R is C₁-C₆alkyl or C₁-C₆alkyl substituted by phenyl, into a 2-sulfenylphenylsulfonamide of the formula IV 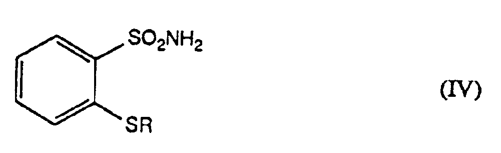 in which R is as defined under formula III,
b) this compound is oxidized to give the 2-sulfinylphenylsulfonamide of the formula V 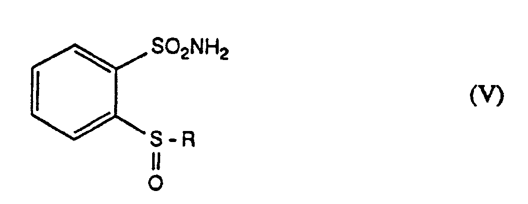 in which R is as defined under formula III,
c) the resulting 2-sulfinylphenylsulfonamide of the formula V is converted, in the presence of an acid, into the disulfide of the formula VI 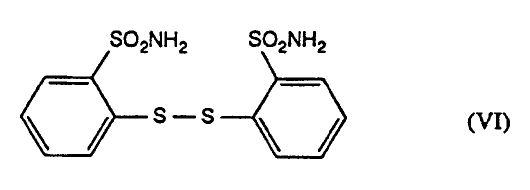
d) the disulfide of the formula VI is reduced to the 2-mercaptophenylsulfonamide of the formula VII 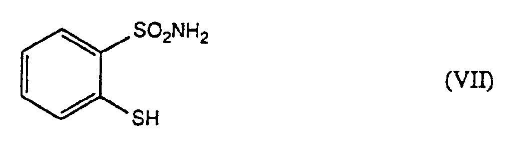
e) this compound is then converted, by means of a trialkylamine of the formula X
(R₁)₃N (X)
in which R₁ is C₁-C₄alkyl, into the 2-mercaptophenylsulfonamide trialkylamine salt of the formula VIII 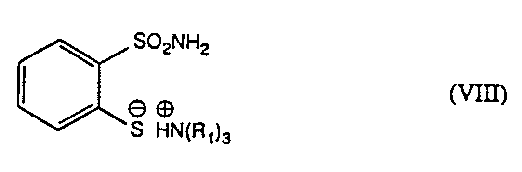 in which R₁ is as defined under formula X, and
f) this compound is then reacted with a halogenofluoroethane of the formula IX
Y-CH₂CFZ₁Z₂ (IX)
in which Y is chlorine or bromine and Z₁ and Z₂ independently of one another are hydrogen, fluorine or chlorine.

2. A process according to claim 1, wherein triethylamine is used as the trialkylamine of the formula X.

3. A process according to claim 1, wherein X is fluorine.

4. A process according to claim 1, wherein R is C₁-C₃alkyl or C₁-C₃alkyl which is substituted by phenyl.

5. A process according to claim 3, wherein R is C₁-C₃alkyl which is substituted by phenyl.

6. A process according to claim 4, wherein R is benzyl.

7. A process according to claim 1, wherein the base used in the reaction of the compound of the formula II with the compound of the formula III is a hydride, hydroxide, carbonate or alcoholate of an alkali or alkaline earth metal, a trialkylamine or a pyridine base.

8. A process according to claim 7, wherein the base used is pyridine, sodium hydroxide, sodium methylate, sodium ethylate, sodium carbonate or potassium carbonate.

9. A process according to claim 7, wherein the base used is potassium carbonate.

10. A process according to claim 1, wherein the oxidizing agent used for the conversion of the compound of the formula IV into the compound of the formula V is hydrogen peroxide, peracetic acid, perbenzoic acid, periodic acid, potassium permanganate or potassium periodate.

11. A process according to claim 10, wherein the oxidizing agent used is hydrogen peroxide.

12. A process according to claim 1, wherein the reducing agent used for the conversion of the disulfide of the formula VI into the 2-mercaptophenylsulfonamide of the formula VII is hydrogen in the presence of platinum, palladium, rhodium or nickel catalysts, or zinc, iron or tin in the presence of hydrochloric acid or acetic acid.

13. A process according to claim 12, wherein the reducing agent used is zinc in the presence of hydrochloric acid or acetic acid.

14. A process according to claim 1, wherein the 2-sulfenylsulfonamide of the formula IV is converted directly into the disulfide of the formula VI without isolation of the intermediate of the formula V.

15. A process according to claim 1, wherein Y is bromine.

16. A process according to claim 1, wherein the reaction of the 2-halogenophenylsulfonamide of the formula II with the mercaptan of the formula III is carried out at a temperature from +20 to +120°C.

17. A process according to claim 1, wherein the oxidation of the 2-sulfenylphenylsulfonamide of the formula IV is carried out at a temperature from 0° to 40°C.

18. A process according to claim 1, wherein the reaction of the 2-mercaptophenylsulfonamide trialkylamine salt of the formula VIII with the 2-halogenofluoroethane of the formula IX is carried out at a temperature from 0° to 80°C.

19. A process according to claim 1, wherein Z₁ is hydrogen and Z₂ is hydrogen, fluorine or chlorine.

20. A process according to claim 1, wherein a) a 2-fluorophenylsulfonamide of the formula II is converted, in the presence of a base and at a temperature from +50 to +70°C, by means of a benzyl mercaptan of the formula III into the 2-benzylthiophenylsulfonamide of the formula IV,
b) this compound is oxidized by means of hydrogen peroxide at a temperature from +5 to +15°C to give the 2-benzylsulfinylphenylsulfonamide of the formula V,
c) this compound is converted in the presence of hydrochloric acid or sulfuric acid into the disulfide of the formula VI,
d) the disulfide of the formula VI is reduced by means of zinc in the presence of acetic acid to give the 2-mercaptophenylsulfonamide of the formula VII,
e) this compound is converted by means of triethylamine into the 2-mercaptophenylsulfonamide triethylamine salt of the formula VIII and
f) this compound is then reacted with 2-bromo-1-fluoroethane, 2-bromo-1,1-difluoroethane, 2-bromo-1-chloro-1-fluoroethane or 1,2-dichloro-1-fluoroethane.

21. A process according to claim 20, wherein the base used is pyridine, sodium hydroxide, sodium methylate, sodium ethylate, sodium carbonate or potassium carbonate.

22. A process according to claim 21, wherein the base used is potassium carbonate.

23. A process according to claim 21, wherein the 2-benzylthiophenylsulfonamide of the formula IV is converted into the disulfide of the formula VI in the presence of hydrochloric acid and hydrogen peroxide without isolation of the intermediate of the formula V.

24. A 2-mercaptophenylsulfonamide trialkylamine salt of the formula VIII

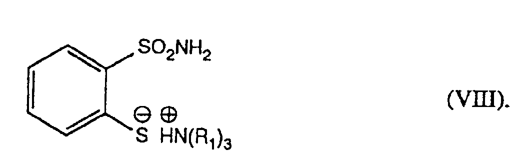

25. A compound of the formula VIII according to claim 24 wherein R₁ is ethyl in each case.

## Revendications

1. Procédé de préparation d'un 2-(2-halogénoéthylthio)-phénylsulfonamide de formule I 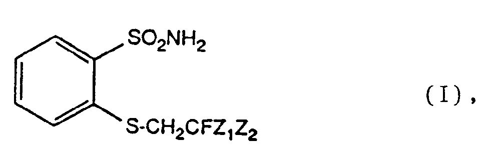 dans laquelle Z₁ et Z₂ représentent chacun, indépendamment l'un de l'autre, l'hydrogène, le fluor ou le chlore, caractérisé en ce que
a) on convertit un 2-halogénophénylsulfonamide de formule II 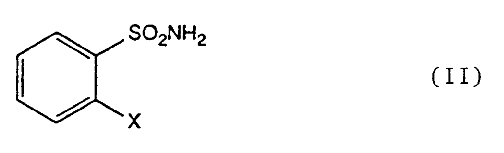 dans laquelle X représente le fluor, le chlore ou le brome, en présence d'une base, à l'aide d'un mercaptan de formule III
R - SH (III)
dans laquelle R représente un groupe alkyle en C1-C6 ou un groupe alkyle en C1-C6 à substituant phényle, en un 2-sulfénylphénylsulfonamide de formule IV 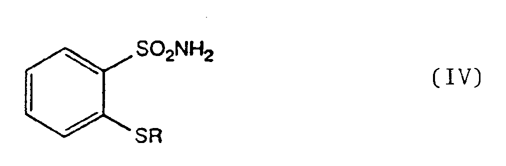 dans laquelle R a les significations indiquées en référence à la formule III,
b) on oxyde ce dernier en le 2-sulfinyl-phénylsulfonamide de formule V 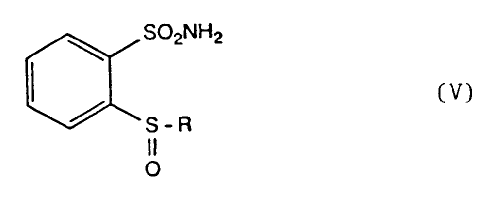 dans laquelle R a les significations indiquées en référence à la formule III,
c) on convertit le 2-sulfinyl-phénylsulfonamide de formule V ainsi obtenu, en présence d'un acide, en le disulfure de formule VI 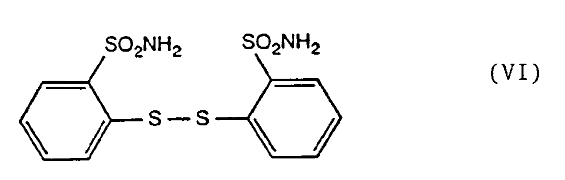
d) on réduit le disulfure de formule VI en le 2-mercapto-phénylsulfonamide de formule VII 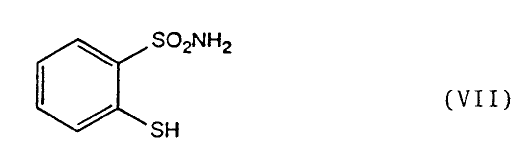
e) on convertit ce dernier, à l'aide d'une trialkylamine de formule X
(R₁)₃N (X)
dans laquelle R₁ représente un groupe alkyle en C1-C4, en le sel de trialkylamine du 2-mercapto-phénylsulfonamide, de formule VIII 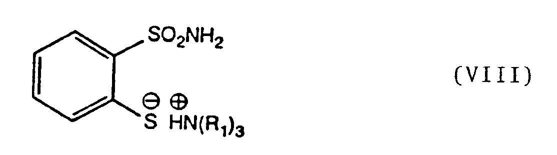 dans laquelle R₁ a les significations indiquées en référence à la formule X et
f) on fait ensuite réagir ce dernier avec un halogéno-fluoréthane de formule IX
Y-CH₂CFZ₁Z₂ (IX)
dans laquelle Y représente le chlore ou le brome et Z1 et Z₂ représentent chacun, indépendamment l'un de l'autre, l'hydrogène, le fluor ou le chlore.

2. Procédé selon la revendication 1, caractérisé en ce que l'on utilise la triéthylamine en tant que trialkylamine de formule X.

3. Procédé selon la revendication 1, caractérisé en ce que X représente le fluor.

4. Procédé selon la revendication 1, caractérisé en ce que R représente un groupe alkyle en C1-C3 ou un groupe alkyle en C1-C3 à substituant phényle.

5. Procédé selon la revendication 3, caractérisé en ce que R représente un groupe alkyle en C1-C3 à substituant phényle.

6. Procédé selon la revendication 4, caractérisé en ce que R représente un groupe benzyle.

7. Procédé selon la revendication 1, caractérisé en ce que, pour la réaction du composé de formule II avec le composé de formule III, on utilise en tant que base un hydrure, un hydroxyde, un carbonate ou un alcoolate d'un métal alcalin ou alcalino-terreux, une trialkylamine ou une base pyridique.

8. Procédé selon la revendication 7, caractérisé en ce que l'on utilise en tant que base la pyridine, l'hydroxyde de sodium, le méthylate de sodium, l'éthylate de sodium, le carbonate de sodium ou le carbonate de potassium.

9. Procédé selon la revendication 7, caractérisé en ce que la base utilisée est le carbonate de potassium.

10. Procédé selon la revendication 1, caractérisé en ce que, pour la conversion du composé de formule IV en le composé de formule V, on utilise en tant qu'agent oxydant le peroxyde d'hydrogène, l'acide peracétique, l'acide perbenzoïque, l'acide periodique, le permanganate de potassium ou le periodate de potassium.

11. Procédé selon la revendication 10, caractérisé en ce que l'agent oxydant utilisé est le peroxyde d'hydrogène.

12. Procédé selon la revendication 1, caractérisé en ce que, pour convertir le disulfure de formule VI en le 2-mercapto-phénylsulfonamide de formule VII, on utilise en tant qu'agent réducteur l'hydrogène en présence de catalyseurs au platine, au palladium, au rhodium ou au nickel, ou bien le zinc, le fer ou l'étain en présence d'acide chlorhydrique ou d'acide acétique.

13. Procédé selon la revendication 12, caractérisé en ce que l'on utilise en tant qu'agent réducteur le zinc en présence d'acide chlorhydrique ou d'acide acétique.

14. Procédé selon la revendication 1, caractérisé en ce que l'on convertit directement le 2-sulfénylsulfonamide de formule IV en le disulfure de formule VI sans isoler le produit intermédiaire de formule V.

15. Procédé selon la revendication 1, caractérisé en ce que Y représente le brome.

16. Procédé selon la revendication 1, caractérisé en ce que la réaction du 2-halogénophénylsulfonamide de formule II avec le mercaptan de formule III est réalisée à une température de +20 à +120°C.

17. Procédé selon la revendication 1, caractérisé en ce que l'oxydation du 2-sulfénylphénylsulfonamide de formule IV est réalisée à une température de 0 à 40°C.

18. Procédé selon la revendication 1, caractérisé en ce que la réaction du sel de trialkylamine du 2-mercapto-phénylsulfonamide de formule VIII avec le 2-halogénofluoréthane de formule IX est réalisée à une température de 0 à 80°C.

19. Procédé selon la revendication 1, caractérisé en ce que Z₁ représente l'hydrogène et Z₂ l'hydrogène, le fluor ou le chlore.

20. Procédé selon la revendication 1, caractérisé en ce que a) on convertit un 2-fluorophénylsulfonamide de formule II, en présence d'une base, à une température de +50 à +70°C, à l'aide d'un benzylmercaptan de formule III, en le 2-benzylthio-phénylsulfonamide de formule IV,
b) on oxyde ce dernier à une température de +5 à +15°C, à l'aide du peroxyde d'hydrogène, en le 2-benzylsulfinyl-phénylsulfonamide de formule V,
c) on convertit ce dernier, en présence d'acide chlorhydrique ou d'acide sulfurique, en le disulfure de formule VI,
d) on réduit le disulfure de formule VI à l'aide du zinc en présence d'acide acétique en le 2-mercaptophénylsulfonamide de formule VII,
e) on convertit ce dernier, à l'aide de la triéthylamine, en le sel de triéthylamine du 2-mercaptophénylsulfonamide de formule VIII et
f) on fait réagir ce sel avec le 2-bromo-1-fluoréthane, le 2-bromo-1,1-difluoréthane, le 2-bromo-1-chloro-1-fluoréthane ou le 1,2-dichloro-1-fluoréthane.

21. Procédé selon la revendication 20, caractérisé en ce que la base utilisée est la pyridine, l'hydroxyde de sodium, le méthylate de sodium, l'éthylate de sodium, le carbonate de sodium ou le carbonate de potassium.

22. Procédé selon la revendication 21, caractérisé en ce que la base utilisée est le carbonate de potassium.

23. Procédé selon la revendication 21, caractérisé en ce que l'on convertit le 2-benzylthiophénylsulfonamide de formule IV, sans isoler le produit intermédiaire de formule V, en présence d'acide chlorhydrique et de peroxyde d'hydrogène, en le disulfure de formule VI.

24. Les sels de trialkylamine du 2-mercapto-phénylsulfonamide, de formule VIII

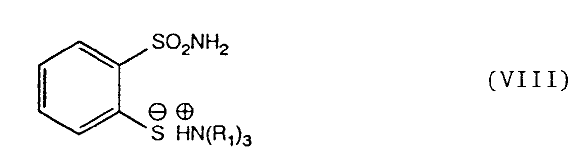

25. Composé de formule VIII selon la revendication 24, caractérisé en ce que tous les symboles R₁ représentent des groupes éthyle.
